# EUROPEAN PATENT SPECIFICATION

(11) **EP 0 954 219 B1**
(45) Date of publication and mention of the grant of the patent: **07.05.2003**
(21) Application number: 98900529.3
(22) Date of filing: 19.01.1998
(51) Int. Cl.: A01K 1/015

(54) **GRID FOR ANIMAL HOUSES**
STALLROST
GRILLES POUR BATIMENTS D'ELEVAGE D'ANIMAUX

(30) Priority: 20.01.1997 DK 7297
(43) Date of publication of application: 10.11.1999
(73) Proprietor: Kongsgaard, Bjarne, 8600 Silkeborg (DK)
(72) Inventor: Kongsgaard, Bjarne, 8600 Silkeborg (DK)
(74) Representative: Nielsen, Leif
(86) International application number: DK9800023
(87) International publication number: WO98031214

(56) References cited:
- EP-A- 0 259 910
- WO-A-95/18525
- DE-A- 3 244 732
- US-A- 4 796 563

## Description

### Background of the invention

The present invention relates to a grating for building up a floor in livestock stables, especially in a pigsty, and comprising an outer, substantially rectangular encircling provided with projecting coupling means and which supports a surface formed of grating bars between which there is gap for the passage of animal droppings, and which is intended to form a floor surface, and which is divided into sections forming different levels in the surface, said sections form a check pattern.

From US A 4,796,563 there is known such a grating. This grating shows sharp transitions between different levels. Therefore, there is a risk of pressure damages on the feet of the animals and risk of collecting animal droppings in corners.

Floor gratings with surfaces divided into sections are known. The sections are divided by beads standing up from the floor surface and dividing this into the sections. It is the purpose of these beads to prevent the animals from slipping and especially it is the intention to prevent that the animals slip when they are about to rise or lay down.

In the known gratings the grating surface within each section may either be plain or curving downwards. Thus, it has been proposed to make the sections curving downwards at least over a part of the extension of the section. Such a downward curving will ensure that the animals step in the middle of the section. This is an disadvantage, because it may damage the hoofs of the animals to step on the beads themselves. This will give rise to very large local pressure loads.

However, it appears that the gratings with the projecting beads have a relatively small self-cleaning ability. Because of the projecting beads, animal droppings may be placed in immediate connection with the beads. Since the animals will not step on and around the beads and since relatively sharp edged comers occur, some of the droppings will collect in the corner between the beads and the top side in the individual sections. Thereby, a relatively large ammonia evaporation from the stable floor will occur.

Furthermore, the beads will give rise to harmful local pressure loads on the animals, when they are lying on the stable floor. When the animal rests with a large weight, a large part of the weight will rest on the raised beads. This gives rise to very strong pressure loads which may cause pressure damages, for example bad blood circulation, bruises, etc.

In certain countries there are requirements for reducing the ammonia evaporation from the stable floor. To fulfil such requirements, it will be necessary to have a stable floor which is self-cleaning to the greatest possible extent, i.e. a stable floor where animal droppings are stepped down in the space between the grating bars by the normal movement of the animals on the stable floor. Such a self-cleaning effect is insufficient with the known stable floors of the kind mentioned in the introduction.

It is the object of the present invention to provide a grating of the kind mentioned in the introduction, where the above-mentioned drawbacks are remedied and which has a self-cleaning ability while at the same time there is no risk of harmful local pressure loads on the animals.

This is achieved according to the present invention with a grating which is characterised in that the sections at least form elevated areas which are wholly surrounded by areas or sections that are at lower levels, that there is a smooth transition between the different levels, and that the sections in transverse direction and longitudinal direction of the check pattern with respect to the sides of the grating are curving downwards and upwards, respectively, against a central section part.

According to a special embodiment, the central section part is substantially plane. There is a smooth transition between the single sections.

A grating of the kind mentioned in the introduction will usually be made of plastic, and therefore has a relatively slippery surface. Alternatively, the grating may also be made of cast iron.

As the grating has elevated areas distributed over the floor surface, the feet of the animals will be prevented from sliding. When a check pattern is used where the check pattern is alternately upwards and downwards curving, it will be ensured with very great certainty that the feet of the animals do not slide. The feet of the animals will normally be placed in the downwards curving sections. The hoofs of the animals will be prevented from sliding, as they will find support at the smooth transition between such a downwards curving section and an upwards curving section. When the animal lies down or rises, it will thus be able to set off against such a transition to an elevated area or a transition between adjoining sections. As a smooth transition between the individual sections occurs, the animal may also step on the area, where the transition between adjoining sections appear, just as the animal may step on the upwards curving sections. If the sections curving upwards are provided with a non-skid cover, there will be achieved a very safe floor, where the risk of sliding is also avoided, even if the animal steps on the upwards curving sections. However, when the animal stands on the floor, there will be no risk of their sliding, even if their hoofs are on an upwards curving section. The problem with sliding on the grating will occur especially when the animal is about to rise or lay down. In this situation, it will not be a problem, when the hoof of the animal slide a distance over the floor, until it finds a point where it can set off.

As the animal thus in a safe way may step on both the elevated areas or in the downwards and in the upwards curving sections and in the transition between the individual sections, it will be ensured with very great certainty that animal droppings are stepped down over all of the surface of the grating. As a smooth transition occurs between the individual sections, there will be no risk of collection of animal droppings in corners or at edges at the surface of the grating, thereby giving rise to ammonia evaporation from the surface of the grating.

When the animal is lying on the floor, its weight will be evenly distributed over the whole floor surface. Even though a greater pressure load will occur in the sections which are upwards curving, there will not occur local pressure loads which can give rise to harmful pressure loads, for example in the shape of the so-called shoulder damages. Because of the smooth transition between the sections, it will also be possible that the body of the animal follows the curvature so that the pressure is distributed substantially over all of the surface without local pressure loads occurring. This will be the case especially if no great difference of height between the lowest and the highest part of the sections in the upwards curving and downwards curving parts occurs. Thus, it is preferred to use differences in height between 5 and 40 mm and preferably between 10 and 15 mm. That means that part elevations between 2½ and 20 mm in the individual sections occur. However, it should be mentioned that the sections do not necessarily have to be symmetrically downwards curving and upwards curving. There may thus occur a greater downwards directed curving and a lesser upwards directed curving. Hereby, it will be easier for the hoofs of the animal to find support in the downwards curving sections while at the same time the risk that the hoofs of the animals slide down the upwards curving parts is reduced.

A suitable size for sections for use in pigsties has turned out to be sections having a substantially quadratic shape with a side length of between 50 and 100 mm. However, it is to be mentioned that the sections may also be rectangular. Furthermore, the sections may be rhombic. Such a rhombic form will occur, when the check pattern is oriented with an angle relative to the sides of the grating. Such an oblique orientation is particularly suitable in a pigsty, where the gratings will normally be placed with an orientation corresponding to the orientation in which the animals normally are. for example in sow pens. When such an animal will rise, it will displace the legs away from itself. In order that a sow pressing its legs out from itself during the rising finds support in the best way, the angle relative to the sides of the grating and thereby relative to the longitudinal direction of the sow may preferably be between 15 and 75°, preferably between 30 and 60°, and especially between 40 and 50°. Because the sow will press its legs away from itself under an angle of about 45°, such an oblique orientation of the check pattern will cause the sow to support itself approximately perpendicularly against a transition between two adjoining sections.

As the grating bars will be oriented in parallel with one of the sides of the grating, and when the check pattern is oriented with an angle relative to the side of the said grating, there is achieved a particularly safe self-cleaning ability. Thus, spaces between grating bars will extend transversely to the transition so that with a particularly great certainty, there is also achieved a stepping down of animal droppings in this area.

### Description of the drawing

The invention will be explained in more detail hereinafter with reference to the attached schematic drawing, in which
- Fig. 1: shows a perspective view of a grating according to the invention as seen from above,
- Fig. 2: shows a view of the grating in Fig. 1 as seen from below,
- Fig. 3: shows a partial view in an enlarged scale of the grating shown in Fig. 1
- Fig. 4: shows a partial view of a second part in an enlarged scale of the grating shown in Fig. 1,
- Fig. 5: shows a view of a further embodiment of a grating according to the invention,
- Fig. 6: shows a view of a further embodiment of a grating according to the invention,
- Fig. 7: shows a section through the grating shown in Fig. 5,
- Fig. 8: shows a section through the grating shown in Fig. 6,
- Fig. 9: shows a view of a further embodiment of a grating according to the invention,
- Fig. 10: shows a view of a further embodiment of a grating according to the invention,
- Fig. 11: shows a section through the grating shown in Fig. 9, and
- Fig. 12: shows a section through the grating shown in Fig. 10.

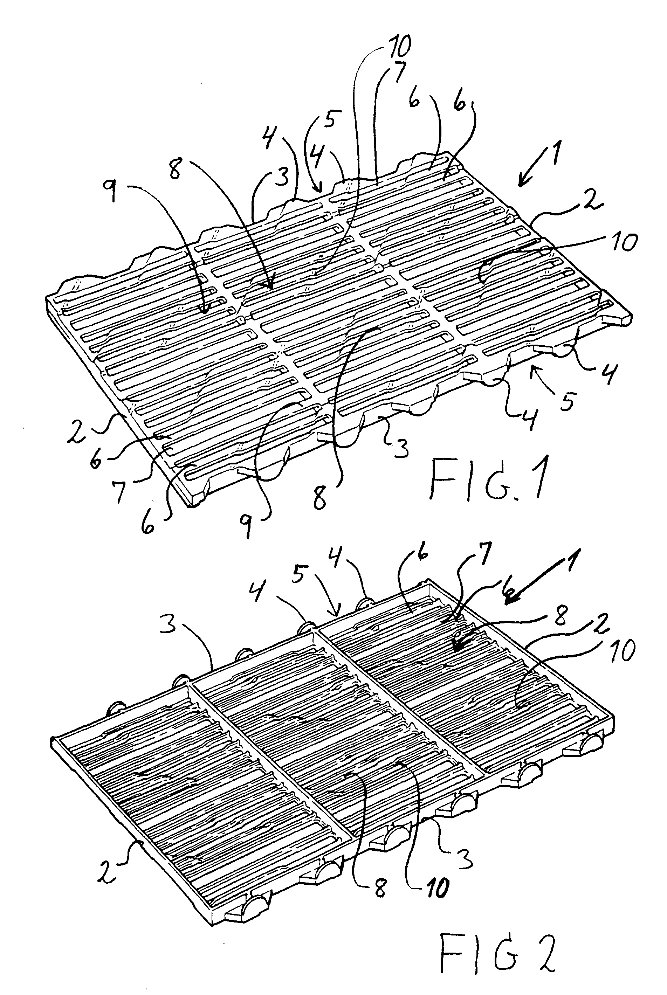

In Fig. 1, there is shown a grating 1 intended for use when building up a floor in livestock stables. The grating comprises an outer substantially rectangular encircling consisting of two end sides 2 and two lateral sides 3. At the lateral sides 3, there are teeth 4 comprising downwards directed grooves for use at the mounting of the grating. Between the teeth 4 there are spaces 5 for receiving teeth for an adjoining grating 1. The gratings may thus be used in a known way to make a floor. The gratings comprise grating bars 6 between which there are gaps 7 intended for passage of animal droppings. The grating bars 6 have a surface forming the floor surface on which the animals are placed. The grating comprises sections so that the floor surface appears with different levels. In Fig. 1 elevated sections 8 are seen. Between the elevated sections 8 there are areas 9 which are at a lower level. The transition 10 between a section 8 and the surrounding areas or sections 9 is a smoothly curved transition.

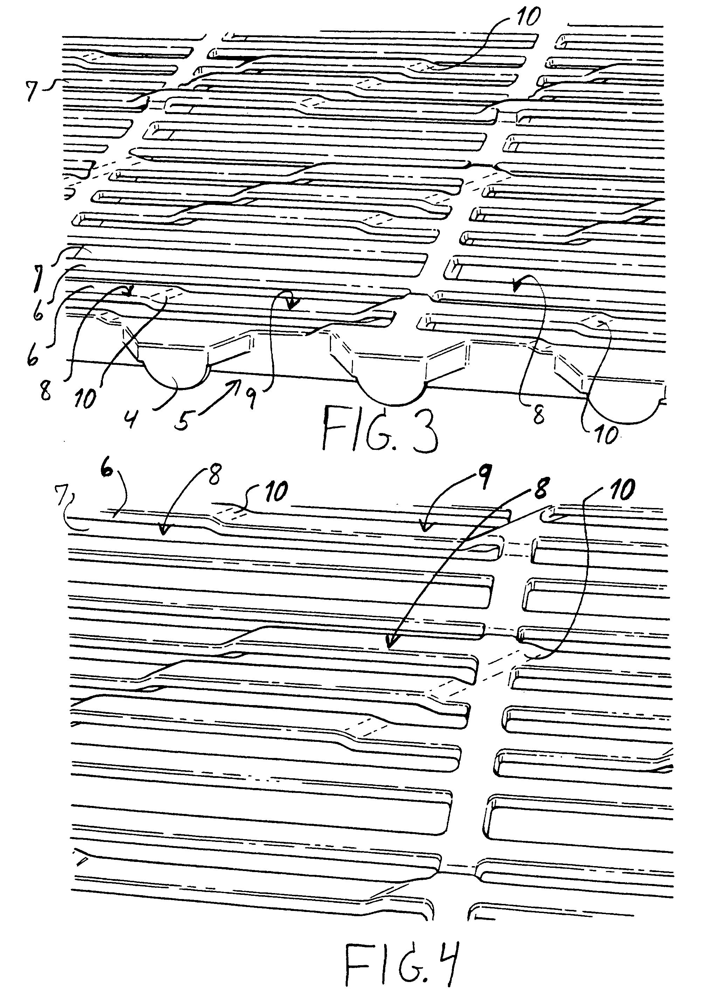

The transition between the different sections is more clearly seen in Figs. 3 and 4. The grating shown in Figs. 1-4 is a kind where elevated sections 8 are completely surrounded by the area 9 which is at a lower level. Within the sections 8, the floor surface appears substantially plane just as the floor surface in the area 9 is also substantially plain.

In the embodiment shown in Figs. 1-4, the grating bars 6 are oriented in parallel with the sides of the grating 3. The check pattern formed by the elevated areas 8 is oriented under an angle relative to said grating side. Each of the check areas appears thus with a substantially rhombic appearance.

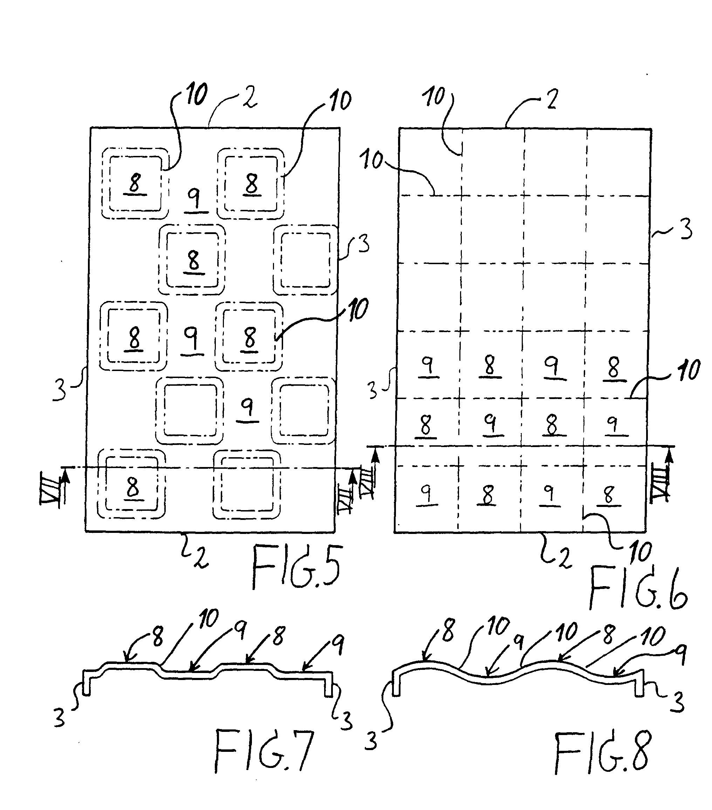

In Fig. 5 another embodiment is shown where elevated sections 8 are completely surrounded by areas 9 which are at the lower level. In this embodiment, the check pattern is oriented in parallel with the sides of the grating 2,3. Each of the check areas 8 thus appear with a substantially rectangular, approximately quadratic, appearance.

In Fig. 6 is seen an embodiment, where sections 8 together with sections 9 form a check pattern. Each of the elevated areas 8 are thus at their comers adjoined to a corresponding elevated area, just as each of the individual areas 9 which are placed at a lower level in each comer will adjoin a corresponding area 9 placed at a lower level.

Of the sections shown in Figs. 7 and 8 it is seen how smooth transitions 10 between an elevated section 8 and an area 9 at a lower level occur. In Fig. 8 it is seen that the sections 8 are curving upwards from the outside against a central upper section part which may be substantially plane just as the sections 9 are curving smoothly downwards against the central section parts which are also mainly plane.

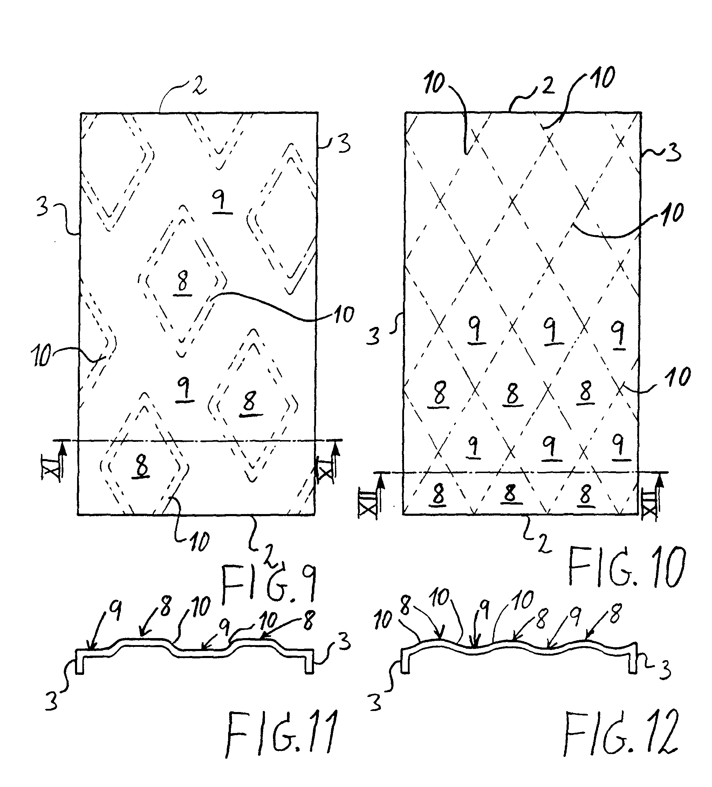

The embodiments shown in Figs. 9 and 10 differ from the embodiments shown in Figs. 5-6 in that the formed sections are rhombic. As the same references are used to denote identical or corresponding elements, there will not be given any specific explanation in connection with the embodiments shown in Figs. 9 and 10 or the sectional views shown in Figs. 11 and 12. However, it should be mentioned that at the top side of each elevated side 8 in Fig. 12, there may be provided a cover (not shown), which for example is of a rubber material in order to make the upwards curving section safe against slipping.

## Claims

1. A grating (1) for building up a floor in livestock stables, especially in a pigsty, and comprising an outer, substantially rectangular encircling (2,3) provided with projecting coupling means (4) and which supports a surface formed of grating bars (6) between which there is gap (7) for the passage of animal droppings, and which is intended to form a floor surface, and which is divided into sections (8,9) forming different levels in the surface, said sections (8,9) form a check pattern, **characterised in that** the sections at least form elevated areas (8) which are wholly surrounded by areas or sections (9) that are at lower levels, that there is a smooth transition (10) between the different levels, and that the sections (8,9) in transverse direction and longitudinal direction of the check pattern with respect to the sides (2,3) of the grating are curving downwards and upwards, respectively, against a central section part.

2. A grating according to claim 1, **characterised in that** the central section part is substantially plane.

3. A grating according to claim 1 or 2, **characterised in that** the coupling means (4) are projecting teeth (4) arranged for interacting with corresponding coupling teeth and with support rails received in a groove at the underside of the teeth.

4. A grating according to claim 1, 2, or 3, **characterised in that** the grating bars (6) are oriented in parallel with one of the sides (3) of the grating, and that the check pattern is oriented at an angle relative to said side (3) of the grating.

5. A grating according to claim 4, **characterised in that** the angle is between 15 and 75°, preferably between 30 and 60°, especially between 40 and 50°.

6. A grating according to claim 1, 2 or 3, **characterised in that** the grating bars (6) are oriented in parallel with one of the sides (3) of the grating, and that the check pattern is oriented in parallel with said side (3) of the grating.

7. A grating according to any of the preceding claims, **characterised in that** the difference of height between the lowest and the highest part of the sections (9,8) is between 5 and 40 mm, preferably between 10 and 15 mm.

8. A grating according to any of the preceding claims, **characterised in that** each section (8,9) has an extension between 50 x 50 mm and 100 x 100 mm.

9. A grating according to any of the preceding claims, **characterised in that** it is made of plastic.

10. A grating according to any of the preceding claims, **characterised in that** the upwardly curving sections (8) are provided with a non-skid cover, preferably one of rubber material.

## Patentansprüche

1. Gitterrost (1) zum Aufbau eines Bodens in Viehställen, insbesondere in einem Schweinestall, und mit einer äußeren, im wesentlichen rechteckigen Umfassung (2, 3), die mit vorstehenden Kupplungseinrichtungen (4) versehen ist und die eine Fläche trägt, die aus Gitterstäben (6) ausgebildet ist, zwischen denen sich eine Lücke (7) für den Durchgang von Tierausscheidungen befindet, und das zur Ausbildung einer Bodenfläche bestimmt ist und das in Abschnitte (8, 9) unterteilt ist, die verschiedene Niveaus in der Oberfläche ausbilden, wobei die Abschnitte (8, 9) ein Schachbrettmuster ausbilden, **dadurch gekennzeichnet, dass** die Abschnitte mindestens erhabene Bereiche (8) ausbilden, die vollständig von Bereichen oder Abschnitten (9) umgeben sind, die auf unteren Niveaus liegen, dass es einen sanften Übergang (10) zwischen den verschiedenen Niveaus gibt und dass sich die Abschnitte (8, 9) in Querrichtung und Längsrichtung des Schachbrettmusters in Bezug auf die Seiten (2, 3) des Gitterrostes nach unten bzw. nach oben gegen einen Zentralabschnittsteil krümmen.

2. Gitterrost nach Anspruch 1, **dadurch gekennzeichnet, dass** der Zentralabschnittsteil im wesentlichen eben ist.

3. Gitterrost nach Anspruch 1 oder 2, **dadurch gekennzeichnet, dass** die Kupplungseinrichtungen (4) vorstehende Zähne (4) sind, die dafür eingerichtet sind, mit entsprechenden Kupplungszähnen und mit Tragschienen zusammenzuwirken, die in einer Rille an der Unterseite der Zähne aufgenommen sind.

4. Gitterrost nach Anspruch 1, 2 oder 3, **dadurch gekennzeichnet, dass** die Gitterstäbe (6) parallel zu einer der Seiten (3) des Gitterrostes orientiert sind und dass das Schachbrettmuster unter einem Winkel relativ zu der Seite (3) des Gitterrostes orientiert ist.

5. Gitterrost nach Anspruch 4, **dadurch gekennzeichnet, dass** der Winkel zwischen 15 und 75°, vorzugsweise zwischen 30 und 60°, insbesondere zwischen 40 und 50° ist.

6. Gitterrost nach Anspruch 1, 2 oder 3, **dadurch gekennzeichnet, dass** die Gitterstäbe (6) parallel zu einer der Seiten (3) des Gitterrostes orientiert sind und dass das Schachbrettmuster parallel zu der Seite (3) des Gitterrostes orientiert ist.

7. Gitterrost nach einem der vorhergehenden Ansprüche, **dadurch gekennzeichnet, dass** die Höhendifferenz zwischen dem niedrigsten und dem höchsten Teil der Abschnitte (9, 8) zwischen 5 und 40 mm, vorzugsweise zwischen 10 und 15 mm beträgt.

8. Gitterrost nach einem der vorhergehenden Ansprüche, **dadurch gekennzeichnet, dass** jeder Abschnitt (8, 9) eine Ausdehnung zwischen 50 x 50 mm und 100 x 100 mm hat.

9. Gitterrost nach einem der vorhergehenden Ansprüche, **dadurch gekennzeichnet, dass** er aus Kunststoff besteht.

10. Gitterrost nach einem der vorhergehenden Ansprüche, **dadurch gekennzeichnet, dass** die nach oben gekrümmten Abschnitte (8) mit einem rutschfesten Belag versehen sind, vorzugsweise einem aus Gummimaterial.

## Revendications

1. Caillebotis (1) destiné à créer un plancher dans les écuries d'élevage, particulièrement dans une porcherie, et comprenant un cerclage (2, 3) extérieur, sensiblement rectangulaire pourvu de moyens de liaison en saillie (4) et qui soutien une surface formée de barres du caillebotis (6) entre lesquelles il y a un espacement (7) pour le passage des déjections des animaux, et qui est prévu pour former une surface de plancher, et qui est divisé en sections (8, 9) formant des niveaux différents dans la surface, lesdites sections (8, 9) forment un damier, **caractérisé en ce que** les sections forment au moins des zones élevées (8) qui sont entièrement entourées par des zones ou sections (9) qui sont à des niveaux inférieurs, qu'il y a une transition douce (10) entre les différents niveaux, et **en ce que** les sections (8, 9) dans le sens transversal et le sens longitudinal du damier par rapport aux côtés (2, 3) du caillebotis sont incurvées vers le bas et vers le haut, respectivement, par rapport à une partie de section centrale.

2. Caillebotis selon la revendication 1, **caractérisé en ce que** la partie de section centrale est sensiblement plane.

3. Caillebotis selon la revendication 1 ou 2, **caractérisé en ce que** les moyens de liaison (4) sont des dents en saillie (4) disposées pour agir réciproquement avec les dents en saillie correspondantes et avec des tubes d'appui reçus dans une rainure au niveau de la surface intérieure des dents.

4. Caillebotis selon la revendication 1, 2 ou 3, **caractérisé en ce que** les barres du caillebotis (6) sont orientées parallèlement à l'un des côtés (3) du caillebotis, et **en ce que** le damier est orienté à un angle par rapport audit côté (3) du caillebotis.

5. Caillebotis selon la revendication 4, **caractérisé en ce que** l'angle est compris entre 15 et 75°, de préférence entre 30 et 60°, surtout entre 40 et 50°.

6. Caillebotis selon la revendication 1, 2 ou 3, **caractérisé en ce que** les barres du caillebotis (6) sont orientées parallèlement à l'un des côtés (3) du caillebotis, et **en ce que** le damier est orienté parallèlement audit côté (3) du caillebotis.

7. Caillebotis selon l'une quelconque des revendications précédentes, **caractérisé en ce que** la différence de hauteur entre la partie la plus basse et la plus élevée des sections (9, 8) est comprise entre 5 et 40 mm, de préférence entre 10 et 15 mm.

8. Caillebotis selon l'une quelconque des revendications précédentes, **caractérisé en ce que** chaque section (8, 9) présente une extension entre 50 x 50 mm et 100 x 100 mm.

9. Caillebotis selon l'une quelconque des revendications précédentes, **caractérisé en ce qu'**il est réalisé en matière plastique.

10. Caillebotis selon l'une quelconque des revendications précédentes, **caractérisé en ce que** les sections incurvées vers le haut (8) sont pourvues d'un revêtement anti-dérapant, de préférence d'un revêtement en caoutchouc.
